# EUROPEAN PATENT SPECIFICATION

(11) **EP 2 992 768 B1**
(45) Date of publication and mention of the grant of the patent: **20.01.2021**
(21) Application number: 14848352.2
(22) Date of filing: 23.01.2014
(51) Int. Cl.: A24F 47/00, A61M 15/06

(54) **ELECTRONIC CIGARETTE**
ELEKTRONISCHE ZIGARETTE
CIGARETTE ÉLECTRONIQUE

(30) Priority: 29.09.2013 CN 201310459545
(43) Date of publication of application: 09.03.2016
(73) Proprietor: Shenzhen Smoore Technology Limited, Shenzhen, Guangdong 518102 (CN)
(72) Inventor: CHEN, Zhiping, Shenzhen Guangdong 518102 (CN)
(74) Representative: Manitz Finsterwald Patent- und Rechtsanwaltspartnerschaft mbB
(86) International application number: PCT/CN2014/071191
(87) International publication number: WO 2015/043133

(56) References cited:
- EP-A1- 2 022 349
- EP-A1- 2 404 515
- EP-A1- 2 460 423
- EP-A1- 2 460 424
- WO-A1-2013/083638
- CN-A- 101 843 368
- CN-U- 201 467 998
- CN-U- 202 276 832
- CN-Y- 201 379 072
- JP-U- 3 164 408
- US-A1- 2002 168 419
- US-A1- 2011 226 236

## Description

### FIELD OF THE INVENTION

The present disclosure relates to an electronic cigarette. The features of the preamble of the independent claim are known from WO 2013/083638 A1. Related technologies are known from EP 2 460 424 A1, EP 2 404 515 A1 and EP 2 022 349 A1.

### BACKGROUND OF THE INVENTION

The electronic cigarette is also known as a virtual cigarette or an electronic atomizer. As a replacement for cigarette, the electronic cigarette is usually used for smoking cessation. The appearance and taste of the electronic cigarette are similar to that of the conventional cigarette, while it does not contain tar, suspended particles and other harmful ingredients as the conventional cigarette.

The electronic cigarette is mainly composed of an atomizer and a power assembly. The atomizer is the core device of the electronic cigarette to generate atomizing gas; the quality and taste of the smoke are depended on the atomization effect. In the conventional atomizer, only the liquid located close to a heating wire can be heated and atomized, while the atomization effect of the liquid located away from the heating wire is poor. Or even if those liquid can be atomized, the atomized particles are relatively large due to the low atomization temperature.

### SUMMARY OF THE DISCLOSURE

The present disclosure is directed to an electronic cigarette, as defined in the independent claim, having a better atomization effect.

An electronic cigarette includes a housing, and an atomizing assembly and a liquid reservoir received in the housing. The housing has an airflow channel formed therein. The liquid reservoir is configured to provide liquid to the atomizing assembly. The atomizing assembly includes a heating tube capable of absorbing liquid, the heating tube defines a plurality of micropores on a wall thereof, a surface of the heating tube is partially exposed to air in the airflow channel.

In the forgoing electronic cigarette, the heating tube has a larger surface area (heating area), and the outer surface of the heating tube is in contact with the liquid reservoir, thus the liquid absorbing area of the heating tube is large. Partial surface of the heating tube is exposed to the air in the airflow channel, such that the smoke produced by atomizing can be well transported to the outside, thus enabling a better atomization effect of the electronic cigarette.

### BRIEF DESCRIPTION OF THE DRAWINGS

The components in the drawings are not necessarily drawn to scale, the emphasis instead being placed upon clearly illustrating the principles of the present disclosure. Moreover, in the drawings, like reference numerals designate corresponding parts throughout the views.
FIG. 1 is a cross-sectional view of an electronic cigarette according to a first embodiment;
FIG. 2 is an exploded, perspective view of the electronic cigarette shown in FIG. 1;
FIG. 3 is a perspective view of the heating tube shown in FIG. 2;
FIG. 4 is a front view of the heating tube shown in FIG. 3 in an expanded state;
FIG. 5 is a perspective view of the heating tube according to a second embodiment;
FIG. 6 is a perspective view of the heating tube according to a third embodiment;
FIG. 7 is a cross-sectional view of the electronic cigarette according to a fourth embodiment;
FIG. 8 is a partial, cross-sectional view of the electronic cigarette according to a fifth embodiment; and
FIG. 9 is a bottom plan view of the heating tube according to a embodiment, where the outer wall of the heating tube is provided with grooves extending axially.

### DETAILED DESCRIPTION OF THE EMBODIMENTS

Illustrative embodiments of the disclosure are below. The following explanation provides specific details for a thorough understanding of and enabling description for these embodiments. One skilled in the art will understand that the disclosure may be practiced without such details. In other instances, well-known structures and functions have not been shown or described in detail to avoid unnecessarily obscuring the description of the embodiments.

Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively. Additionally, the words "herein,""above," "below" and words of similar import, when used in this application, shall refer to this application as a whole and not to any particular portions of this application. When the claims use the word "or" in reference to a list of two or more items, that word covers all of the following interpretations of the word: any of the items in the list, all of the items in the list and any combination of the items in the list.

### Example One

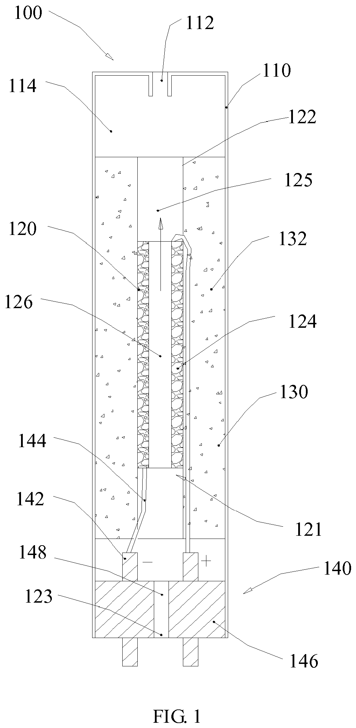

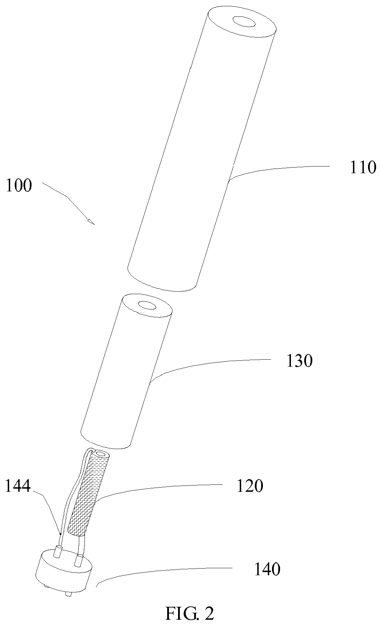

Referring to FIG. 1 and FIG. 2, a first embodiment of an electronic cigarette 100 includes a housing 110, a liquid reservoir 130, a power supply assembly 140, and an atomizing assembly 121. The liquid reservoir 130, the power supply assembly 140, and the atomizing assembly 121 are received in the housing 110. The power supply assembly 140 is used to supply power to the atomizing assembly 121.

The housing 110 is made of plastic, which is approximately shaped as a cylinder. The housing 110 is hollow and has a cavity for receiving various internal components of the electronic cigarette 100. In alternative embodiments, the housing 110 may have a shape of a rectangular cylinder, oval cylinder and so on. The housing 110 defines an air outlet 112 on an end thereof and an air intake 123 on the other end thereof. The housing 110 forms an airflow channel 122 therein in fluid communication with the air outlet 112 and the air intake 123. The housing 110 is provided with a filter nozzle (not shown) close to the end of the air outlet 112 to filter nicotine and other chemical substances in the smoke.

The liquid reservoir 130 includes storage medium such as temperature resistance and non-toxic sponge, thus the liquid can be absorbed and stored in the liquid reservoir 130. The liquid reservoir 130 has a receiving channel 125 extending along an axial direction, partial inner wall of the receiving channel 125 surrounds the outer wall of the heating tube 120, and the receiving channel 125 forms part of the airflow channel 122. In addition, the liquid reservoir 130 and the air outlet 112 define a buffer space 114 therebetween, and the filter nozzle is located in the buffer space 114.

The atomizing assembly 121 includes a heating tube 120. The heating tube 120 has a channel 126, which also forms part of the airflow channel 122, such that the inner surface of the heating tube 120 is exposed to the air in the airflow channel 122. The heating tube 120 defines a plurality of micropores 124 which penetrates the wall of the heating tube 120. A density of the micropores 124 on the heating tube 120 is 1200 to 4900 micropores per square centimeter. Partial inner surface of the receiving channel 125 is attached to partial outer surface of the heating tube 120. Accordingly, the liquid stored in the liquid reservoir 130 can be directly absorbed by the heating tube 120 by capillary action, and the liquid is free to crawl on the heating tube 120 and pass through the micropores 124 on the wall of the heating tube 120. The liquid can be uniformly heated to form uniformly atomized particles. Besides, the heating area of the heating tube 120 is enlarged due to the configuration of the micropores 124, and the contact area between the heating tube 120 and the liquid is also enlarged, the speed of the atomization is accelerated.

The heating tube 120 has a much larger heating area due to its tubular shape. A proper amount of the liquid can be evenly heated, and a uniform particles and a suitable amount of smoke are generated, thus the taste is great. In addition, the fabrication process of the heating tube 120 is relatively simple. In the assembly process of electronic cigarette 100, there is no need to conduct the cumbersome process of twining a heating wire around a fixed axis, thereby the assembly operation is simplified. The flow of the liquid can be adjusted by adjusting the contact area between the inner surface of the liquid reservoir 130 and the outer surface of the heating tube 120, therefore the risk that the atomization effect is effected by too much or too less liquid absorbed by the heating tube 120 is avoided.

In one embodiment, partial liquid reservoir 130 surrounds the outer surface of the heating tube 120, such that when the liquid reservoir 130 provides liquid for the heating tube 120, the heat transferred from the heating tube 120 to the housing 110 is reduced. The heat loss of the heating tube 120 is reduced and the efficiency of the heat of the heating tube 120 is improved. Furthermore, the risk that the housing is thermal deformed or even harming to the consumers can be avoided. When the liquid reservoir 130 is used as a container, it can also function as a support for the heating tube 120, thus simplifying the inner structure of the electronic cigarette 100. What is much important is that, the amount of the mounting components in the housing 110 is reduced, the space for the air circulation is enlarged, such that the consumers can easily inhale the smoke.

The heating tube 120 has the channel 126 through which air in the airflow channel 122 can pass, such that the liquid in the heating tube 120 can reach the inner surface by passing through the heating tube 120, and the atomized liquid can be exhausted by the channel 126 and enters the airflow channel 122, the space for the air circulation in the housing 110 is further enlarged, such that the consumers can easily inhale the smoke.

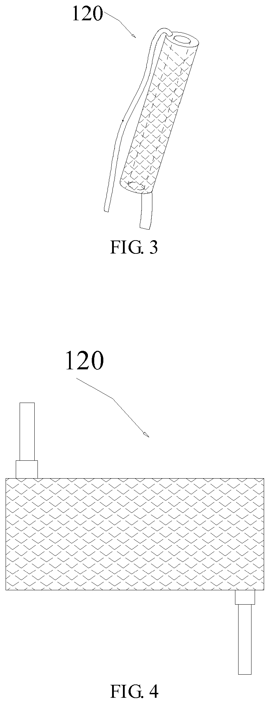

The structure of the heating tube 120 is shown in FIG. 3. In the illustrated embodiment, the heating tube 120 is a mixed woven tube 220 composed of flexible heating strips and heating resistant fibers. The mixed woven tube 220 is hollow and has a microporous structure itself, the impurities in the liquid can pass through the micropores to be removed from the mixed woven tube 220, or it can be absorbed by the heating resistant fibers which have a greater adsorption capacity, thereby the impurities is not likely to be remained on the flexible heating strips of the mixed woven tube 220 to affect the heating effect. The flexible heating strips may be metal wires or conductive fibers and the like, as long as it can be woven. The heating resistant fibers can be glass fibers or carbon fibers with temperature resistance. The flexible heating strips are mixed with the heating resistant fibers to form a tubular heating surface with greater superficial area. The absorption capacity of the heating resistant fibers is great, the absorption area is also great, and the flexible heating strips are sufficiently in contact with heating resistant fibers, such that the atomization effect is great. The FIG. 4 is a front view of the mixed woven tube 220 in an expanded state, it can be seen that the mixed woven tube 220 has a grid-like structure. The fabrication process of the mixed woven tube 220 is highly targeted and has a strong variability. Compared to the heating tube of the conventional electronic cigarette, the mixed woven tube 220 can be automated produced, and the production difficulty is reduced. The process parameters such as electric resistance are also easier to be controlled.

Referring to FIG. 1 and FIG. 2 again, the power supply assembly 140 is electrically connected to the heating tube 120 to supply power to the heating tube 120. In the illustrated embodiment, the power supply assembly 140 includes an electrode 142, a wire 144, an electrode support 146, and battery (not shown). The electrode 142 is connected to the heating tube 120 by the wire 144. The electrode 142 is inserted into the electrode support 146, the electrode support 146 defines an air hole 148 for air-circulate. Alternatively, the electrode support 146 can be omitted.

The assembly process of the electronic cigarette 100 is described as follows: first of all, the liquid reservoir 120 is fixed in the cavity of the housing 110, and the heating tube 120 is connected to the electrode 142 of the power supply assembly 140 by the wire 144, then the electrode 142 is inserted into the electrode support 146, finally, the housing 110 is sleeved to the electrode support 146 and the assembly is completed. It should be understood that the assembly sequence can be adjusted as needed.

In use, as the arrows shown in the FIG. 1, when the user inhales the electronic cigarette 100 on the end of the air outlet 112, the airflow enters the electronic cigarette 100 from the air intake 123, then passes through the air hole 148 of the electrode support 146, and carries the atomized liquid to the airflow channel 122, and finally enters the mouth of the user by passing through the air outlet 112.

Example Two - which does not fall under the scope of the appended claims and thus is not part of the claimed invention.

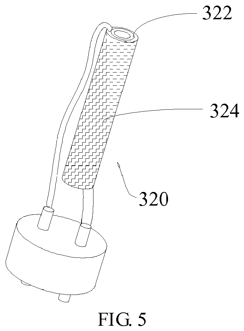

Referring to FIG. 5, the heating tube 320 is similar to the heating tube 120 of the first embodiment, the difference is that: the heating tube 320 includes a substrate 322 and a conductive paint-coating layer 324. The substrate 322 is made of a material having an internal microporous structure, and 2000 to 3000 micropores are disposed per square centimeter on the heating tube 120. The substrate 322 has a hollow tubular shape. The conductive paint-coating layer 324 is coated on surface of the substrate 322. In the illustrated embodiment, the conductive paint-coating layer 324 is a conductive heating paint-coating layer. Painting conductive heating high-temperature painting material onto the substrate 322 to form the conductive heating paint-coating layer. Further, the conductive heating high-temperature painting material can be painted on both the inner and outer surfaces of the substrate 322 to increase the heating area. The conductive heating high-temperature painting material are painting material including carbon black, aluminum, aluminum dihydrogen phosphate. In alternative examples, the conductive heating high-temperature painting material may be conductive particles painting material containing metal fibers and carbon fibers, plastics and the conductive carbon particles, or metal powders and the other conductive particles, etc. There is no need to twine a heating wire around the fixed axis to form the heating tube 320, thereby the assembly operation is simplified and the cost is down. The material of the heating tube 320 is more environmentally friendly, and the resistance is relatively stable.

Example Three - which does not fall under the scope of the appended claims and thus is not part of the claimed invention.

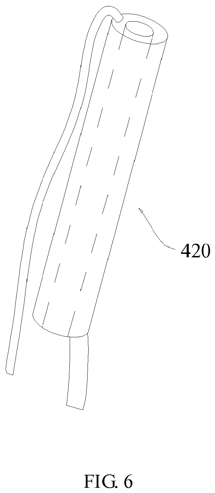

Referring to FIG. 6, the heating tube 420 is similar to the heating tube 120 of the first embodiment, the difference is that: the heating tube 420 is made of foamed metal, foamed graphite or porous ceramic. The heating tube 420 has a plurality of internal micropores formed by extruding or firing, thus the heating tube 420 can produce heat by itself. The foamed metal, foamed graphite or porous ceramic exhibits the functions of heating and absorbing liquid, the absorbed liquid is atomized when heating. Moreover, the foamed metal, foamed graphite or porous ceramic possesses the properties of high electrical conductivity and thermal conductivity, and also has the characteristics of large specific surface area, completely through holes, high porosity, lightweight, and easy processing. Besides, the foamed metal, foamed graphite or porous ceramic has a certain strength and rigidity, the fixed device such as the fixed axis can be omitted, thereby the structure and assembly operation are simplified. Moreover, the heating tube 420 can be fabricated by using a standard mold, thus the resistance and size can be easy adjusted. In the illustrated embodiment, the heating tube 420 is made of foamed nickel-chromium.

### Example Four

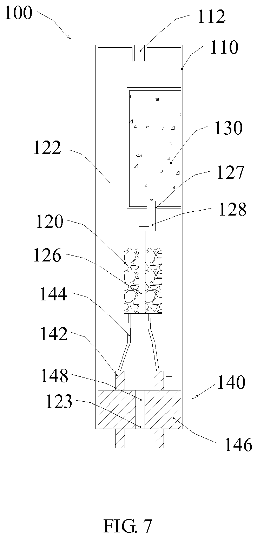

Referring to FIG. 7, the electronic cigarette is similar to that of the first embodiment, the difference is that: the channel 126 of the heating tube 120 forms no part of the airflow channel 122, instead, it is formed by a gap between the heating tube 120 and the housing 110. The outer surface of the heating tube 120 is exposed to the air in the airflow channel 122, thereby the air in the airflow channel 122 is capable of blowing through the outer surface of the heating tube 120. The liquid reservoir 130 is fixed to the housing 110. The liquid reservoir 130 is a sealed container surrounded by partial wall of the housing 110 and is used for store the liquid. The liquid reservoir 130 is shaped approximately as a cylinder. In the illustrated embodiment, a cross-sectional area of the liquid reservoir 130 is less than that of the housing 110, and a gap between the liquid reservoir 130 and an inner wall of the housing 110 forms the airflow channel 122 . The liquid reservoir 130 defines a liquid outlet 127 at an end thereof. One end of a liquid conductor 128 is connected to a liquid outlet 127 of the liquid reservoir 130, and the other end thereof extends inside the channel 126 of the heating tube 120.

The liquid conductor 128 is made of sponge with great capacity of absorbing liquid, therefore not only the liquid stored in the liquid reservoir 130 can be transported by capillary action, but the phenomenon that the heating tube 120 is directly in contact with the liquid reservoir 130 can be avoided, thus the amount of heat transferring to the liquid reservoir 130 is reduced. Accordingly, the use efficiency of the heat is greatly increased, and the risk that the liquid reservoir 130 is accelerated aged because the liquid reservoir 130 is heated for the long time by the heating tube 120 can be avoided, and the powder generated by the liquid reservoir 130 which can affect the quality of the smoke can also be avoided. Moreover, the damage of the liquid reservoir 130 caused by the heating tube 120 can be avoided. The flowing air in the housing 110 can be in contact with the outer surface of the heating tube 120 and bring out the atomized liquid on the outer surface of the heating tube 120.

### Example Five

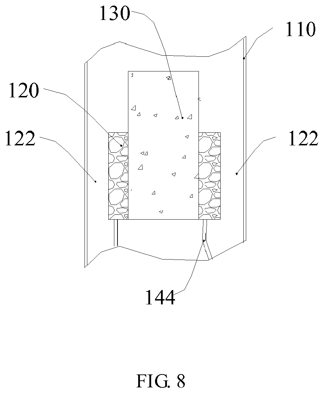

Referring to FIG. 8, the electronic cigarette is similar to that of the first embodiment, the channel 126 of the heating tube 120 forms no part of the airflow channel 122, the heating tube 120 surrounds partial outer surface of the liquid reservoir 130. The airflow channel 122 is located outside of the heating tube 120, thereby the outer surface of the heating tube 120 is exposed to the air in the airflow channel 122, the air in the airflow channel 122 is capable of blowing through the outer surface of the heating tube 120. Such configuration can solve the problem of large doses of atomization of the liquid.

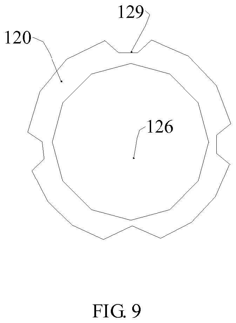

Furthermore, referring to FIG. 9, the heating tube 120 further defines a groove 129 extending along an axial direction on an outer wall thereof. Alternatively, the heating tube 120 can define a groove 129 extending along an axial direction on an inner wall thereof. The contact area between the liquid reservoir 130 and the heating tube 120 can be adjusted by the groove 129, and the groove 129 can also form an assisted airflow channel to enhance the gas flux.

Although the subject matter has been described in language specific to structural features, it is to be understood that the subject matter defined in the appended claims is not necessarily limited to the specific features described above. Rather, the specific features described above are disclosed as example forms of implementing the claims. Numerous modifications and alternative arrangements can be devised without departing from the scope of the invention as defined in the claims.

## Claims

1. An electronic cigarette (100), comprising:
a housing (110) having an airflow channel (122) formed therein;
a liquid reservoir (130) received in the housing (110) and configured to provide liquid to an atomizing assembly (121); and
the atomizing assembly (121) received in the housing (110), wherein the atomizing assembly (121) comprises a heating tube (120) capable of absorbing liquid, the heating tube (120) defines a plurality of micropores (124) on a wall thereof and penetrating the wall, a surface of the heating tube (120) is partially exposed to air in the airflow channel (122),
**characterized in that**
the heating tube (120) is a mixed woven tube (220) composed of flexible heating strips and heating resistant fibers, wherein the flexible heating strips are mixed with the heating resistant fibers.

2. The electronic cigarette according to claim 1, wherein the liquid reservoir (130) surrounds at least part of the outer wall of the heating tube (120), the air in the airflow channel (122) is capable of passing through the heating tube (120).

3. The electronic cigarette according to claim 2, wherein an inner sidewall of the liquid reservoir (130) is attached to the wall of the heating tube (120).

4. The electronic cigarette according to claim 1, wherein the heating tube (120) defines a groove (129) extending axially on the wall thereof.

5. The electronic cigarette according to claim 1, wherein the heating tube (120) surrounds at least a part of the liquid reservoir (130), the air in the airflow channel (122) is capable of being blown passing an outer surface of the heating tube (120).

6. The electronic cigarette according to claim 5, wherein the wall of the heating tube (120) is attached to the outer sidewall of the liquid reservoir (130).

7. The electronic cigarette according to claim 1, further comprising a liquid conductor (128), wherein an end of the liquid conductor (128) is connected to the liquid reservoir (130), and the other end of the liquid conductor (128) extends inside the heating tube (120).

8. The electronic cigarette according to claim 1, wherein the liquid reservoir (130) is a container having an opening formed by surrounding partial sidewall of the housing (110).

9. The electronic cigarette according to claim 1, wherein a density of the micropores (124) on the heating tube (120) is 1200 to 4900 micropores per square centimeter.

10. The electronic cigarette according to claim 1, wherein the liquid reservoir (130) defines a receiving channel (125) extending along an axial direction, a part of the inner wall of the receiving channel (125) surrounds an outer wall of the heating tube (120).

11. The electronic cigarette according to claim 1, wherein a cross-sectional area of the liquid reservoir (130) is less than a cross-sectional area of the housing (110); and the airflow channel (122) is formed between a sidewall of the liquid reservoir (130) and a sidewall of the housing (110).

12. The electronic cigarette according to claim 1, wherein the airflow channel (122) is located in a center of the housing (110).

## Patentansprüche

1. Elektronische Zigarette (100), umfassend:
ein Gehäuse (110) mit einem darin ausgebildeten Luftstromkanal (122);
ein Flüssigkeitsreservoir (130), das in dem Gehäuse (110) aufgenommen und so konfiguriert ist, dass es Flüssigkeit für eine Zerstäubungsanordnung (121) bereitstellt; und
die Zerstäubungsanordnung (121), die in dem Gehäuse (110) aufgenommen ist, wobei die Zerstäubungsanordnung (121) ein Heizrohr (120) umfasst, das in der Lage ist, Flüssigkeit zu absorbieren, das Heizrohr (120) eine Vielzahl von Mikroporen (124) auf einer Wand davon definiert und die Wand durchdringt, eine Oberfläche des Heizrohrs (120) teilweise der Luft in dem Luftstromkanal (122) ausgesetzt ist,
**dadurch gekennzeichnet, dass**
das Heizrohr (120) ein gemischt gewebtes Rohr (220) ist, das aus flexiblen Heizstreifen und hitzebeständigen Fasern besteht, wobei die flexiblen Heizstreifen mit den hitzebeständigen Fasern gemischt sind.

2. Elektronische Zigarette nach Anspruch 1, wobei das Flüssigkeitsreservoir (130) mindestens einen Teil der Außenwand des Heizrohrs (120) umgibt, wobei die Luft im Luftstromkanal (122) durch das Heizrohr (120) hindurchgelangen kann.

3. Elektronische Zigarette nach Anspruch 2, wobei eine innere Seitenwand des Flüssigkeitsreservoirs (130) an der Wand des Heizrohres (120) befestigt ist.

4. Elektronische Zigarette nach Anspruch 1, wobei das Heizrohr (120) eine Nut (129) definiert, die sich axial an seiner Wand erstreckt.

5. Elektronische Zigarette nach Anspruch 1, wobei das Heizrohr (120) mindestens einen Teil des Flüssigkeitsreservoirs (130) umgibt, wobei die Luft im Luftstromkanal (122) an einer Außenfläche des Heizrohrs (120) vorbei geblasen werden kann.

6. Elektronische Zigarette nach Anspruch 5, wobei die Wand des Heizrohrs (120) an der äußeren Seitenwand des Flüssigkeitsreservoirs (130) befestigt ist.

7. Elektronische Zigarette nach Anspruch 1, ferner mit einem Flüssigkeitsleiter (128), wobei ein Ende des Flüssigkeitsleiters (128) mit dem Flüssigkeitsreservoir (130) verbunden ist und das andere Ende des Flüssigkeitsleiters (128) sich in das Innere des Heizrohrs (120) erstreckt.

8. Elektronische Zigarette nach Anspruch 1, wobei das Flüssigkeitsreservoir (130) ein Behälter mit einer Öffnung ist, die durch eine umgebende Teilseitenwand des Gehäuses (110) gebildet ist.

9. Elektronische Zigarette nach Anspruch 1, wobei eine Dichte der Mikroporen (124) auf dem Heizrohr (120) 1200 bis 4900 Mikroporen pro Quadratzentimeter beträgt.

10. Elektronische Zigarette nach Anspruch 1, wobei das Flüssigkeitsreservoir (130) einen Aufnahmekanal (125) definiert, der sich entlang einer axialen Richtung erstreckt, wobei ein Teil der Innenwand des Aufnahmekanals (125) eine Außenwand des Heizrohrs (120) umgibt.

11. Elektronische Zigarette nach Anspruch 1, wobei eine Querschnittsfläche des Flüssigkeitsreservoirs (130) kleiner ist als eine Querschnittsfläche des Gehäuses (110); und der Luftstromkanal (122) zwischen einer Seitenwand des Flüssigkeitsreservoirs (130) und einer Seitenwand des Gehäuses (110) ausgebildet ist.

12. Elektronische Zigarette nach Anspruch 1, wobei der Luftstromkanal (122) im Zentrum des Gehäuses (110) angeordnet ist.

## Revendications

1. Cigarette électronique (100), comprenant :
un boîtier (110) à l'intérieur duquel est formé un canal d'écoulement d'air (122) ;
un réservoir de liquide (130) reçu dans le boîtier (110) et configuré pour fournir du liquide à un ensemble d'atomisation (121) ; et
l'ensemble d'atomisation (121) reçu dans le boîtier (110), dans laquelle l'ensemble d'atomisation (121) comprend un tube chauffant (120) capable d'absorber un liquide, le tube chauffant (120) définit une pluralité de micropores (124) sur une de ses parois et pénétrant dans la paroi, une surface du tube chauffant (120) est partiellement exposée à l'air dans le canal d'écoulement d'air (122),
**caractérisée en ce que**
le tube chauffant (120) est un tube tissé mixte (220) composé de bandes chauffantes flexibles et de fibres résistantes à la chaleur, dans laquelle les bandes chauffantes flexibles sont mélangées avec les fibres résistantes à la chaleur.

2. Cigarette électronique selon la revendication 1, dans laquelle le réservoir de liquide (130) entoure au moins une partie de la paroi extérieure du tube chauffant (120), l'air dans le canal d'écoulement d'air (122) est capable de passer à travers le tube chauffant (120).

3. Cigarette électronique selon la revendication 2, dans laquelle une paroi latérale intérieure du réservoir de liquide (130) est fixée à la paroi du tube chauffant (120).

4. Cigarette électronique selon la revendication 1, dans laquelle le tube chauffant (120) définit une rainure (129) s'étendant axialement sur sa paroi.

5. Cigarette électronique selon la revendication 1, dans laquelle le tube chauffant (120) entoure au moins une partie du réservoir de liquide (130), l'air dans le canal d'écoulement d'air (122) est capable d'être soufflé en passant sur une surface extérieure du tube chauffant (120).

6. Cigarette électronique selon la revendication 5, dans laquelle la paroi du tube chauffant (120) est fixée à la paroi latérale extérieure du réservoir de liquide (130).

7. Cigarette électronique selon la revendication 1, comprenant en outre un conducteur de liquide (128), dans laquelle une extrémité du conducteur de liquide (128) est reliée au réservoir de liquide (130) et l'autre extrémité du conducteur de liquide (128) s'étend à l'intérieur du tube chauffant (120).

8. Cigarette électronique selon la revendication 1, dans laquelle le réservoir de liquide (130) est un contenant ayant une ouverture formée par la paroi latérale partielle du boîtier (110) qui l'entoure.

9. Cigarette électronique selon la revendication 1, dans laquelle la densité des micropores (124) sur le tube chauffant (120) est de 1200 à 4900 micropores par centimètre carré.

10. Cigarette électronique selon la revendication 1, dans laquelle le réservoir de liquide (130) définit un canal de réception (125) s'étendant dans une direction axiale, une partie de la paroi intérieure du canal de réception (125) entoure une paroi extérieure du tube chauffant (120).

11. Cigarette électronique selon la revendication 1, dans laquelle une surface de section transversale du réservoir de liquide (130) est inférieure à une surface de section transversale du boîtier (110); et le canal d'écoulement d'air (122) est formé entre une paroi latérale du réservoir de liquide (130) et une paroi latérale du boîtier (110).

12. Cigarette électronique selon la revendication 1, dans laquelle le canal d'écoulement d'air (122) est situé au centre du boîtier (110).
